(19) **Europäisches Patentamt / European Patent Office / Office européen des brevets**

(11) **EP 2 544 547 B1**

(12) # EUROPEAN PATENT SPECIFICATION

(45) Date of publication and mention of the grant of the patent:
**18.06.2014 Bulletin 2014/25**

(51) Int Cl.:
*C12N 1/20* (2006.01)   *A23C 9/123* (2006.01)
*A23C 13/16* (2006.01)   *A23C 15/12* (2006.01)
*A23C 19/06* (2006.01)   *A23L 1/00* (2006.01)
*A23L 1/03* (2006.01)   *A23L 1/30* (2006.01)
*C12N 1/04* (2006.01)

(21) Application number: **11717733.7**

(22) Date of filing: **09.03.2011**

(86) International application number:
**PCT/IB2011/000500**

(87) International publication number:
**WO 2011/110926 (15.09.2011 Gazette 2011/37)**

(54) **FOOD PRODUCT COMPRISING PROBIOTIC BACTERIA COATED WITH A COATING OF VEGETABLE ORIGIN**

NAHRUNGSMITTELPRODUKT MIT PROBIOTISCHEN BAKTERIEN UND EINEM ÜBERZUG PFLANZLICHEN URSPRUNGS

PRODUIT ALIMENTAIRE CONTENANT DES BACTÉRIES PROBIOTIQUES RECOUVERTES D'UN REVÊTEMENT D'ORIGINE VÉGÉTALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.03.2010 IT MI20100390**

(43) Date of publication of application:
**16.01.2013 Bulletin 2013/03**

(73) Proprietor: **Probiotical S.p.A.**
**28100 Novara (IT)**

(72) Inventors:
• **MOGNA, Giovanni**
**I-28100 Novara (IT)**
• **STROZZI, Gian Paolo**
**I-28100 Novara (IT)**
• **MOGNA, Luca**
**I-28100 Novara (IT)**

(74) Representative: **HOFFMANN EITLE**
**Patent- und Rechtsanwälte**
**Arabellastrasse 4**
**81925 München (DE)**

(56) References cited:
JP-A- 8 242 763      US-A- 4 332 790
US-A1- 2003 109 025   US-A1- 2005 002 874
US-A1- 2007 098 847   US-A1- 2008 102 275

• DATABASE FSTA [Online] INTERNATIONAL FOOD INFORMATION SERVICE (IFIS), FRANKFURT-MAIN, DE; 2007, JI-KANG JUNG ET AL: "Survival of double-microencapsulated Bifidobacterium breve in milk in simulated gastric and small intestinal conditions.", XP002591712, Database accession no. FS-2007-08-Pa2013 & JOURNAL OF FOOD SCIENCE AND NUTRITION, vol. 12, no. 1, 2007, page 58,
• SIUTA-CRUCE P ET AL: "IMPROVING PROBIOTIC SURVIVAL RATES MICROENCAPSULATION PRESERVES THE POTENCY OF PROBIOTIC MICROORGANISMS IN FOOD SYSTEMS", FOOD TECHNOLOGY, INSTITUTE OF FOOD TECHNOLOGISTS, CHICAGO, IL, US, vol. 55, no. 10, 1 October 2001 (2001-10-01), pages 36,38-40,42, XP001108329, ISSN: 0015-6639

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a food product comprising probiotic bacteria coated with a coating of vegetable origin. In particular, the present invention relates to a dairy product selected from among milk, cheese, butter, margarine, yogurt and cream comprising probiotic bacteria coated with at least one coating of vegetable origin.

It is well known that there are present on the market food products, e.g. chocolate or yogurt, comprising probiotic bacteria.

However, said products have a number of disadvantages which limit their use.

A first disadvantage relates to the stability of the bacteria within the food product.

In practical terms, lactic bacteria immersed in a food product suffer from a lack of stability, which causes a decline in the bacterial count over time.

The initial declared bacterial count for a given product declines over time because of the lack of stability of the bacteria within it.

Therefore, the initial quantity of bacteria present in the starting product does not correspond, after a certain relatively brief lapse of time after the time of manufacture, to the one declared on the label, because of the decline that occurs in the bacterial count.

A second disadvantage relates to the nature of the food product itself (pH value, free water content, chemical composition from a qualitative and quantitative point of view) which, besides influencing the state of vitality of the bacteria, may condition their efficacy once administered into the body (in vivo viability and functionality).

Finally, there are also several considerations to be made on the stability of the food product itself. In practical terms, after a certain interval of time, lactic bacteria immersed in a food product may give rise to precipitation and/or aggregation phenomena with the subsequent formation of a bacterial aggregate or precipitate. These phenomena can alter the shelf life of the food product.

US 4332790 discloses a microcapsule containing a microorganism in which the microorganism is of the genus Lactobacillus and/or Bifidobacterium and/or other geni of the intestinal flora coated with a fat which is solid at body temperature.

US 2007/098847 relates to a liquid and aqueous food product containing dehydrated lactic bacteria granules and coated with a vegetable fat material.

US 2005/002874 relates to new strains of Lactobacillus that have been selected for their capability to reduce the number of Streptococcus mutans in the mouth of mammals through inhibiting activity in combination with better binding to the oral mucins and dental plaque, thereby preventing, reducing or treating dental caries, and products derived from said strains, including agents for treatment or prophylaxis of caries for administration to humans.

US 2003/109025 concerns particles containing dehydrated living micro-organisms coated with a homogeneous layer of hydrophobic substance selected to reduce the risks of degradation of said micro-organisms by physico-chemical stresses such as heat, humidity, gastric acid, or compression. The invention also concerns a method for producing such particles, which consists in injecting a melting hydrophobic substance into the mass of said dehydrated micro-organisms placed in a chamber swept by an air stream at controlled temperature and whereof the base is rotating. The invention further concerns the particles obtainable by said method, and the use of said particles in pharmaceutical, dietetic or food compositions.

JP H08242763 refers to a yogurt having good flavor and excellent in quality stability and functionality by adding three-layered capsules produced by covering a useful enteral bacterium as an inner content with an inner coating film comprising an edible substance and with an enteric outer coating film to the yogurt.

JI-KANG JUNG ET AL: "Survival of double-microencapsulated Bifidobacterium breve in milk in simulated gastric and small intestinal conditions.", XP002591712, Database accession no. FS-2007-08-Pa2013 & JOURNAL OF FOOD SCIENCE AND NUTRITION, vol. 12, no. 1, 2007, page 58, relates to the survival of double-microencapsulated Bifidobacterium breve in milk, in simulated gastric and small intestinal conditions.

SIUTA-CRUCE P ET AL: "IMPROVING PROBIOTIC SURVIVAL RATES MICROENCAPSULATION PRESERVES THE POTENCY OF PROBIOTIC MICROORGANISMS IN FOOD SYSTEMS", FOOD TECHNOLOGY, INSTITUTE OF FOOD TECHNOLOGISTS, CHICAGO, IL, US, vol. 55, no. 10, 1 October 2001 (2001-10-01), pages 36,38-40,42, XP001108329, relates to methods of improving probiotic survival rates.

Therefore, there remains a need to have a food product comprising probiotic bacteria which has improved stability compared to the products available on the market.

In particular, there remains a need to be able to prepare a dairy food product containing probiotic bacteria, in which the initial quantity of bacteria present is not subject to an excessive decline over time leading to a drastic reduction therein. Finally, it is necessary for the food product containing the probiotic bacteria to be prepared in such a way as to maintain the bacteria in a good state of vitality and functionality.

The Applicant has responded to the above-mentioned needs by developing a food product comprising at least one probiotic microorganism, as set forth in the appended independent claim.

Preferred embodiments of the present invention are set forth in the detailed description.

Table 1 shows a list of microorganisms which have valid application in the context of the present invention.

Table 2 shows the stability values of the strains *Bifidobacterium breve* DSM 16604 (BR03) and *Bifidobacterium lactis* LMG P-21384 (BS01) in coated and uncoated form in UHT whole milk after 4 and 7 days of storage at 4 °C. Each strain was introduced into the full cream milk in a quantity such as to have a bacterial count per litre of milk of approximately 6 billion (BLN), so as to guarantee 1 BLN in a 168 ml glass of milk. The stability test was conducted at 4 °C. The shelf life of this product is estimated to be about 7 days.

Table 3 shows the stability values of the strain *Lactobacillus rhamnosus* ATCC 53103 (LGG) in coated and uncoated form in grated Grana Padano cheese, after 134 days of storage at 5 °C. The shelf life of this product is estimated to be about 68 days.

Table 4 shows the stability values of the strain *Lactobacillus rhamnosus* ATCC 53103 (LGG) in coated and uncoated form in butter, after 134 days of storage at 5 °C. The shelf life of this product is estimated to be about 90 days.

Table 5 shows the stability values of the strain *Bifidobacterium breve* DSM 16604 (BR03) in coated and uncoated form in Grana Padano cheese, after 127 days of storage at 5 °C.

Table 6 shows the stability values of the strain *Bifidobacterium breve* DSM 16604 (BR03) in coated and uncoated form in Soresina butter, after 127 days of storage at 5 °C.

Table 7 shows the stability values of the strain *Lactobacillus rhamnosus* ATCC 53103 (LGG) in coated and uncoated form in grated Bella Lodi grana-type cheese, after 120 days of storage at 5 °C.

Table 8 shows the stability values of the strain *Bifidobacterium breve* DSM 16604 (BR03) in coated and uncoated form in grated Bella Lodi grana-type cheese, after 120 days of storage at 5 °C.

Table 9 shows the stability values of the strain *Bifidobacterium breve* DSM 16604 (BR03) and *Bifidobacterium lactis* LMG P-21384 (BS01) in coated and uncoated form in UHT whole milk and in cream, after 4, 15 and 30 days of storage at 4 °C. A quantity of the lyophilized strains was introduced into the milk and cream such as to have a count per litre of product of approximately 6 billion (BLN).

The stability test was conducted at a temperature of +4 °C. The shelf life of the UHT milk is estimated to be about 90 days at room T (closed container) and 3-4 days for an open container stored in the refrigerator. The shelf life of the cream is 1 month for a closed container, stored in the refrigerator, and 3-4 days for an open container stored in the refrigerator.

Table 10 shows the data of the test of table 9 (same strains and same quantities and operating conditions). Furthermore, for each analysis the pH of milk and cream in unopened packages was evaluated in parallel.

Table 11 shows the stability values of the strain *Bifidobacterium breve* DSM 16604 (BR03) and *Bifidobacterium lactis* LMG P-21384 (BS01) in coated and uncoated form in UHT whole milk, after 4, 7, and 15 days of storage at 4 °C. Each strain was introduced into the full cream milk in a quantity such as to have a bacterial count per litre of milk of approximately 6 billion (BLN), so as to guarantee 1 BLN in a 168 ml glass of milk. The stability test was conducted at 4 °C. The shelf life of this product is estimated to be about 90 days at a room temperature of 25 °C.

Table 12 shows the stability values of the strain *Bifidobacterium breve* DSM 16604 (BR03) and *Bifidobacterium lactis* LMG P-21384 (BS01) in coated and uncoated form in pasteurised whole milk after 4, 7, and 15 days of storage at 4 °C. Each strain was introduced into the full cream milk in a quantity such as to have a bacterial count per litre of milk of approximately 6 billion (BLN), so as to guarantee 1 BLN in a 168 ml glass of milk. The stability test was conducted at 4 °C. The shelf life of this product is estimated to be about 4-5 days at 4 °C.

The food product is a dairy product. The dairy product is selected from the group comprising milk, cheese, butter, margarine, yogurt or cream. The cheese is selected from among processed cheese, spreadable cheese, grated cheese, for example Grana Padano and Parmigiano Reggiano, and crescenza and stracchino cheeses. The milk is selected from among pasteurised whole milk and UHT whole milk. The cream may be sour cream for whipping.

The probiotic microorganisms are coated with at least one coating of vegetable origin.

Probiotic microorganisms are live bacteria capable of assuring a beneficial effect to consumers when ingested in large quantities for a sufficient time.

The probiotic bacteria used in the preparation of the dairy product in accordance with present invention are selected from the group comprising the species: *L. acidophilus, L. crispatus, L. gasseri,* group *L. delbrueckii, L. salivarius, L. casei, L. paracasei,* group *L. plantarum, L. rhamnosus, L. reuteri, L. brevis, L. buchneri, L. fermentum, B. adolescents, B. angulatum, B. bifidum, B. breve, B. catenulatum, B. infantis, B. lactis, B. longum, B. pseudocatenulatum* and *S. thermophilus.*

In a preferred embodiment of the invention, the dairy product comprises from one to six strains, preferably from two to four strains, even more preferably three strains selected from among the above-mentioned probiotic species. The probiotic microorganisms used are coated with at least one coating of vegetable origin.

In a preferred embodiment, the probiotic microorganisms are coated with only one coating of vegetable origin.

In another preferred embodiment, the probiotic microorganisms are coated with two coatings of vegetable origin.

Table 1 shows, by way of example, a group of microorganisms which have valid application in the context of the present invention.

All of the strains have been deposited in accordance with the Budapest Treaty and are made accessible to the public, on request, by the competent Depositing Authority.

The probiotic bacteria coated with one or two coatings can be in solid form, in particular in the form of powder, granules, or dehydrated or lyophilized powder.

In the context of the present invention the probiotic bacteria coated with at least one coating of vegetable origin can also be considered as probiotic bacteria microencapsulated with a coating of vegetable origin. In practical terms, the bacteria are coated or microencapsulated with substances of vegetable origin using techniques and processes known to those persons skilled in the art.

For example, the bacteria in lyophilized form can be coated or microencapsulated using a fluid bed technique (e.g. top spray or bottom spray), in which the coating substances, represented by lipids of vegetable origin, are applied on the bacteria.

According to the present invention, the probiotic bacteria are coated (microencapsulated) with at least one coating of vegetable origin, and said at least one lipid coating consists of polyglyceryl-6-distearate.

The coated probiotic bacteria are then added, using known techniques, to the dairy product of the present invention.

The dairy product is selected from the group consisting of milk, cheese, butter, margarine, yogurt and cream. For example, the coated probiotic bacteria in solid form are gradually added, under stirring, to the dairy product, so as to avoid the formation of lumps and agglomerations. Once the addition of bacteria has ended, the dairy product is maintained under stirring for a time comprised from 1 to 20 minutes at a temperature comprised from 4 to 18 °C.

In another embodiment of the present invention, the bacteria added to the dairy product are in the form of coated (microencapsulated) bacteria.

In a preferred embodiment, the lipids of vegetable nature are selected from the group comprising saturated vegetable fats having a melting point comprised from 35 °C to 75 °C, preferably comprised from 45 to 65 °C. Advantageously from 50 to 60 °C.

In a preferred embodiment, saturated vegetable fats having a certain degree of hydrophilicity can be used, which can be selected from among mono- and di-glycerides of saturated fatty acids, polyglycerols esterified with saturated fatty acids, and free saturated fatty acids.

The saturated fatty acids can be selected from the group comprising from 8 to 32 carbon atoms, preferably from 12 to 28 carbon atoms, even more preferably from 16 to 24 carbon atoms.

Advantageously, the coating lipid is selected from the group comprising polyglyceryl distearate (commercial name Plurol Stearique WL 1009), glyceryl palmitostearate (commercial name Precirol Ato 5), saturated fatty acids (commercial name Revel C), hydrogenated vegetable fats of non-lauric origin, and hydrogenated palm fats or stearin.

In a first embodiment, the probiotic bacteria are coated with a single coating (mono-coated). In practical terms, a single coating with a same lipid is realized.

The single coating consists of polyglyceryl-6 distearate CAS 61725-93-7 (commercial name Plurol Stearique WL1009). The mono-coated bacteria are added to the milk, cheese, butter or cream.

In a preferred embodiment, the ratio by weight between lyophilized microorganism and the lipid coating material which coats it is 50:50 or 40:60.

Advantageously, the vegetable lipids in the context of the present invention are carefully selected for their chemical and physical properties. In fact, the coating of the cells (single or double coating) must ensure better sealing of the bacteria from the environment, producing a continuous film without pores communicating with the outside. However, this wrapper must open at the intestinal level to release the bacteria and allow them to colonise. The selected lipids are resistant to acid pH's, so that the coating remains intact in the stomach, but sensitive to even slightly basic pH's, so as to allow the formation of holes in the coating during their passage through the intestine.

The dairy product can contain the coated bacteria in a quantity comprised from 0.1 to 10% by weight; even more preferably in a quantity comprised from 1.5 to 5% by weight, relative to the total weight of the product.

A preferred embodiment of the present invention relates to pasteurised whole milk and UHT milk. The concentration of the coated bacteria is comprised from $1\times10^9$ to $10\times10^9$ CFU/litre of product, preferably from $4\times10^9$ to $7\times10^9$ CFU/litre of product. The bacteria coated with one coating or with two coatings, once added to the milk, give rise to a stable suspension and do not precipitate.

Advantageously, once coated the bacteria added to the food product do not cause any change in the flavour or in the taste of the product itself during the entire shelf life at the product storage temperature.

The subject matter of the present invention moreover relates to the use of coated probiotic bacteria, with a single coating, as described above, for the preparation of a dairy product selected from the group comprising milk, cheese, butter, margarine, yogurt and cream.

The stability values were evaluated using the following counting method.

The counting method allows the bacteria that are coated with a lipid coating to be determined, for the purpose of determining the percentage of coated bacteria, the percentage of bacterial survival and the decline in the bacterial count.

To determine the percentage of bacterial survival in a final product, obtained using a procedure in which the bacteria, for example in lyophilized form, are placed in contact with a lipid, it is necessary to have:

a)A method for determining the "viable count" of the "naked" [a], i.e. uncoated, lyophilizate, also called starting sample. The result is expressed as the number of viable cells per unit of weight of the lyophilizate, typically as the number of billions of Colony-Forming Units (CFU) per gram of product.

b)A method for determining the "total count" of the bacteria after encapsulation/coating [c] which includes the coated bacteria [g] and the uncoated bacteria [b]. The uncoated bacteria comprise free cells and partially coated cells. Thus [c] = [g] + [b]. The result is expressed as the number of viable cells per unit of weight of the lyophilizate, typically as the number of billion of Colony-Forming Units (CFU) per gram of product.

c) Determine the dilution factor (d) which intervenes during encapsulation/coating, given by the ratio between the initial weight of the "naked" lyophilizate (w) and the final weight of the bacteria at the end of the encapsulation process (e), which will be given by the sum of the weight of the "naked" lyophilizate (a) and of the weight of the coating matrix (f). Thus,

$$(w) + (f) = (e); \quad (d) = (w) / (e)$$

The % of survival is given by the "total count" per gram of the bacteria in encapsulated form/viable count per gram of "naked", i.e. uncoated, lyophilizate, also called starting sample, multiplied by the dilution factor (d). The expression of the result as a percentage is possible after multiplication of the aforesaid fraction X 100.

$$\% \text{ of survival} = \{([\text{coated bacteria}] + [\text{uncoated bacteria}]) / \text{viable count of "naked" sample x dilution factor}\} \text{ x } 100;$$

in the formula:

$$\{([c] / [a] \text{ x } [d]) \text{ x} 100\}.$$

The viable count of the "naked", i.e. uncoated, lyophilizate also called starting sample, is determined with a number of methods known to a person skilled in the art.

For the count of the genus *Lactobacillus*, the reference methods used are:

FIL IDF 149A:1997, FIL-IDF 122C:1996, EN-ISO 6887-1 For the count of the genus *Bifidobacterium* the reference methods are: Bulletin of the IDF 252 (3.1.2.iv and viii), EN-ISO 6887-1

These methods enable counting of the viable cells present in the "naked" uncoated lyophilizate, also called starting sample, as is known to those persons skilled in the art.

To determine the percentage of coated bacteria, in contrast, it is necessary to know an additional parameter, namely the count of uncoated bacteria per gram of sample [b], as well as a method for determining it. Following an encapsulation process, in fact, an encapsulated product may comprise bacteria coated with a lipid coating and uncoated bacteria; the latter comprise free cells and partially coated cells.

The uncoated bacteria per gram of sample, indicated with [b], comprise free cells and partially coated cells. To count said uncoated bacteria [b] a known method is used. The use of a saline solution for the bacterial count, for example a peptonated saline solution containing a peptone, for example a casein peptone, sodium chloride and distilled water, enables the determination of the quantity of uncoated bacteria that are present in an encapsulated product. The quantity of uncoated bacteria comprises in turn free cells and partially coated cells.

However, this datum [b] is not useful, on its own, for determining the percentage of coated bacteria unless the number representing the total count of the bacteria [c] present in the coated product is also available.

The percentage of coated bacteria = {(([coated bacteria] + [uncoated bacteria])-[uncoated bacteria]}/ total count of the bacteria after the coating process, the whole multiplied x 100; in the formula: {([g] + [b] - [b]) / [c]} } x100, or {[g]/[c]}x100.

It should be noted that the expression set forth as the numerator in the percentage of coated bacteria "{ ([coated bacteria] + [uncoated bacteria]) - [uncoated bacteria]}", i.e. ([c] - [b]) or, more simply, [g], represents the "total count of bacteria actually coated or encapsulated with a lipid coating" following the encapsulation process.

In the event that the bacteria are placed in contact with a lipid, the total count of the bacteria [c] in the encapsulated product can be determined with a method known in the art which uses lipase at pH 7 (Method NGD C46 - 1976, pp. 1-3 "Determinazione della percentuale di acido palmitico in posizione 2 nei trigliceridi" (*Determination of the percentage of palmitic acid in position 2 in triglycerides); Norme Italiane per il controllo dei grassi e derivati NGD (Italian Standards for the testing of fats and derivatives),* 3rd edition - 1976 and updates, section C46-89).

Solely by way of example, pancreatic lipase is prepared by *Pseudomonas fluorescens* (Aldrich code 39044-5). In practical terms, 1 g of lipase is added to 8.5 g of sodium chloride and then suspended again in 1000 ml of distilled water. The peptonated saline solution has a final pH of 7.0 ± 0.2.

The method which uses lipase at pH 7 is also called M2, enzymatic method, in the context of the present invention.

However, said method that uses lipase at pH 7, applied for the count of the total number of bacteria present in the coated/encapsulated product, has the following disadvantages.

A first disadvantage is given by the fact that lipase is an extremely costly enzyme and, therefore, its use for routine analysis is very limited.

A second disadvantage is given by the fact that lipase, in order to hydrolyze the lipid matrix, needs to be able to act at temperatures comprised from 30 to 40 °C for times comprised from 10 to 20 minutes. In these operative conditions, the coated bacteria do not survive or, in any case, their viability is strongly impaired. The Applicant has noted that the highest mortality affects the fraction of uncoated bacteria [b], since they are more vulnerable to the unfavourable conditions. As a consequence, there is an unpredictable alteration in the ratios [g] / [b] and [g] / [c], and hence of the % of encapsulation.

A third disadvantage is given by the fact that a very accurate control of the reaction time parameter is required since at a temperature comprised from 30 to 40 °C, a small variation of 1-3 minutes will result in an increase in bacterial mortality.

The counting method used enables the determination of the total bacterial count of an encapsulated product.

In particular, it enables the determination of the total bacterial count [c] of an encapsulated product comprising bacteria coated with a lipid coating and uncoated bacteria, the latter comprising free cells and partially coated cells.

Even more in particular, it enables the determination of the percentage of survival, the percentage of coated bacteria and the decline in the bacterial count over time after the bacteria have been placed in contact with a coating lipid.

With the present counting method, the Applicant is capable of determining the total quantity of bacteria [c], expressed as CFU/g of sample, which is present in a sample that comprises bacteria coated with a lipid coating [g] and uncoated bacteria [b], where said sample was obtained by coating a quantity of a starting sample of bacteria (w) with a quantity of lipid (f).

The analytic method of the Applicant serves to overcome the limits of the counting method, known in the background art, which uses the enzyme lipase.

The method enables the determination of the total quantity of bacteria [c] which is present in a sample that comprises bacteria coated with a lipid coating [g] and uncoated bacteria [b].

Moreover, the method enables the determination of the % of bacterial survival in a final product (e) which has been obtained by coating a quantity of a starting sample of bacteria (w) with a quantity of lipid (f).

Finally, the method enables the determination of the percentage of coated bacteria present in a sample that comprises bacteria coated with a lipid coating [g] and uncoated bacteria [b].

All of the scientific articles and methods cited must be considered integrally incorporated into the present description for ready reference.

The method used is a chemical rather than enzymatic method and allows the total quantity of bacteria, which is present in a sample that comprises bacteria coated with a lipid coating and uncoated bacteria, to be determined using a reagent solution.

The method used enables the determination of the total bacterial count after the process of encapsulation of the bacteria with a lipid matrix [c]. Said total count [c] comprises the coated bacteria [g] and the uncoated bacteria[b], the latter comprising free cells and partially coated cells, in the formula [c]=[g] + [b].

The reagent solution is a buffer solution. The reagent solution is an aqueous solution having a pH buffered to a value comprised in the interval from 7 to 9. The reagent solution does not involve the use of lipase. The buffer solution preferably consists of borate buffer which has a pH value that can vary from 7.9 ± 0.1 to 8.4 ± 0.1 at a temperature of 25 ± 2 °C and pressure of 1 atmosphere, in water.

The 0.2 M sodium borate stock solution is prepared, for example, from 12.404 g of boric acid which is added to 100 ml of 1N sodium hydroxide, bringing the final volume to 1 litre. Thereafter, 46.6 ml of 0.1 N HCl solution is added to 53.4 ml of 0.2 M sodium borate stock solution so as to obtain 100 ml of borate buffer solution. The buffer solution is sterilised in an autoclave at 121±1 °C for 15 minutes. The borate buffer solution has a final pH value equal to 8.0 ± 0.1.

The method used involves the use of a buffered reagent solution, which enables the determination of the total count/total quantity of bacteria [c] which is present in an encapsulated/coated product comprising bacteria coated with a lipid coating and uncoated bacteria, the latter comprising free cells and partially coated cells.

The sample to be analysed can contain lactic bacteria and/or bifidobacteria and can be a sample in solid form.

Following the encapsulation process, the bacteria can be completely and/or partially enveloped in the matrix. This will depend on the type of encapsulation, the type of technique used, the device used and, finally, the operating efficiency.

Therefore, in a preferred embodiment of the present invention, the solid product is in anhydrous form and the bacteria, for example lactic bacteria and/or bifidobacteria, are, completely and/or partially, coated by a matrix comprising at least one substance of lipid nature, for example at least one lipid of vegetable and/or animal origin sensitive to a pH value greater than or equal to 7; preferably sensitive to a pH value comprised in the interval from 7 to 9; advantageously to a pH value comprised in the interval from 7.5 to 8.5.

The lipid can comprise an ester between a fatty acid or a mixture of fatty acids and a primary alcohol or a mixture, for example a fatty alcohol or a mixture; the fatty acid itself or a mixture of fatty acids; an ester between a fatty acid or a mixture of fatty acids and an alcohol having two alcohol groups (-OH) or a mixture, an ester between a fatty acid or a mixture and an alcohol having three hydroxyl groups, for example glycerol. The triglyceride can be monoester, di-ester or tri-ester. The fatty acids are selected with a number of carbon atoms greater than 14, preferably comprised from 14 to 22; particularly preferred, from 16 to 18. Palm-derived fatty acids are preferred.

The lipid can be hydrogenated, partially hydrogenated, for example at least 90% hydrogenated, preferably 95% hydrogenated. Alternatively, said lipid can collaterally contain at least one unsaturated fatty acid, preferably monounsaturated. The lipid can have a melting point below 80 °C, preferably comprised from 45 to 65 °C, advantageously from 55 to 60 °C.

In a preferred embodiment, the lipid can be a hydrogenated palm fat having a melting point of approximately 60 °C, a glyceryl dipalmitostearate having a melting point of about 57-60 °C, or a mixture of the two in a ratio by weight comprised from 5:1 a 1:1, preferably 3:1.

For example, the encapsulated product in solid form can be obtained from lactic bacteria and/or bifidobacteria which are lyophilized and submitted to an encapsulation procedure, preferably a microencapsulation procedure, so as to obtain a lyophilizate in which the bacteria have been microencapsulated with a lipid matrix, preferably of vegetable origin, sensitive to a pH value greater or equal to 7 and resistant to a pH value of less than 7. The counting method of is indicated as M1.

The method (M1) for determining the total quantity of bacteria [c], expressed as CFU/g of sample, which is present in a sample that comprises bacteria coated with a lipid coating [g] and uncoated bacteria [b], where said sample has been obtained by coating a quantity of a starting sample of bacteria (w) with a quantity of lipid (f), can comprise the following steps.

A homogeneous solution is prepared comprising a given quantity of said sample to be analysed and a reagent solution.

To prepare said homogeneous solution a dilution of the sample to be analysed (primary dilution) is carried out in order to obtain a diluted sample. Subsequently, a step of homogenising the diluted sample is carried out.

In a preferred embodiment, said primary dilution of a sample to be analysed, for example a sample lyophilizate containing bacteria coated with a lipid coating and uncoated bacteria, is conducted as follows segue:

1. Allow the sample to be analysed thermostat at a temperature, for example, of 25±2 °C for 1 to 2 hours, if it was stored in a freezer, or for at least 30 minutes, if it was stored at a temperature comprised, for example, between 3 and 6 °C.

2. Weigh, for example, about 3-4 g of sample to be analysed in a sterile Stomacher bag.

3. Add the reagent solution in such a way as to obtain a 1:10 primary dilution of the sample to be analysed.

The reagent solution is a buffer solution having characteristics as described above. The reagent solution is an aqueous solution containing the borate buffer.

The sample diluted by means of the primary dilution is submitted to homogenisation. Homogenisation of the diluted sample (suspension) is carried out as indicated in standard UNI EN ISO 6887-1.

Once the primary dilution has been carried out as described above, the sample is homogenised in the buffer solution, for example in a borate buffer solution, for example for a time comprised from 1 to 10 minutes, preferably 5, using a Stomacher homogeniser at 100-500 rpm. As is well known to those persons skilled in the art, this step serves to render the sample to be analysed homogeneous and thus ensure an accurate and repeatable analysis.

At the end of the primary dilution and of the homogenisation step, a suspension is obtained comprising a given quantity of said sample to be analysed and said reagent solution.

The above-mentioned homogeneous suspension is submitted to a dilution step (successive serial dilutions as indicated in standard UNI EN ISO 6887-1), following the methods described hereunder, in order to obtain a diluted solution containing microorganisms.

The successive serial dilutions, indicated with Sn, are in a number comprised from 1 to 20 such as to obtain a suspension having a quantity of CFU/ml comprised from 5 to 400, preferably from 10 to 300, even more preferably from 50 to 200.

A sample consisting of 1 ml of the homogeneous suspension prepared as above is transferred into 9 ml of peptonated saline solution, as described hereunder, so as to obtain the first dilution of the starting homogeneous solution. The peptonated saline solution comprises (pre-sterilisation pH of $7 \pm 0.2$ at 25 °C): NaCl 8.5 g; Bacto peptone 1 g; Water 1000 ml.

The successive decimal dilutions are obtained by transferring, each time, 1 ml of the most concentrated dilution into 9 ml of diluent using a 1 ml graduated sterile pipette, according to the teachings known to those persons skilled in the art.

After a serial dilution is transferred to the successive one, the test tube containing the latter must be agitated by means of a Vortex mixer. 3 agitation cycles are carried out, each of which should last for a time comprised between 5 and 10 seconds, with a sufficient interval of time in between to let the previous vortex come to an end.

The decimal dilutions of the sample are used to determine the titre of active viable cells of a bacterium.

As is well known to those persons skilled in the art, this step is important for the microbiological analysis. In practical terms, the sample containing the bacteria must be suitably diluted in order to determine the appropriate dilution for growing in dishes a number of cells comprised between 10 and 300 cells or bacteria, more preferably from 50 to 200.

For example, if the sample contains a number of cells equal to 200 BLN/g (= $200 \times 10^9$ CFU/g), it is necessary to carry out at least eight serial dilutions of the first dilution, obtained by directly reconstituting the starting powder sample. The eighth serial dilution, for example, will contain 200 live cells/ml of sample, a suitable quantity for counting the colonies in dishes. In this case, it is advisable also to carry out a ninth serial dilution, which is foreseen to contain 20 live cells/ml.

Subsequently, after the successive serial dilutions, an inoculation of the sample is carried out and the culture medium is added to said inoculated sample.

The inoculation of the sample into Petri dishes takes place using the pour plate method, as is well known to those persons skilled in the art, and is briefly described here below solely by way of example:

1. Using a sterile pipette, 1 ml, for example, of the suitable dilutions of the sample is transferred into a sterile Petri dish. The dilutions are selected in such a way as to permit growth in the dish of a number of colonies comprised, for example, between 10 and 300.

2. Subsequently, $12 \pm 1$ ml, for example, of the agarized medium best suited to the bacterial genus and/or species to be counted is added after being melted by means of a microwave oven and subsequently cooled to a temperature, for example, of $45 \pm 0.5$ °C.

A person skilled in the art knows the media described in the official methods.
The known media are:

i) for the growth of *Lactobacilli,* MRS agar (deMan, JC, Rogosa M, and Sharpe ME, J. Applied Microbiology 1960, 23: 130), whereas

ii) for the growth of *Bifidobacteria* two types of medium are indicated:
iia) Rogosa agar (Rogosa M, Mitchell JA, and Wiseman RF, J. Bacteriology, 1951, 62: 132), and
iib) TPY agar (Scardovi, 1981).

The Applicant adopted the medium LAPTg agar for the growth of both bacterial species. For the preparation of the medium see LAPTG: FONT DE VALDEZ, G, and coll.: Influence of the recovery medium on the viability of injured freeze-dried lactic acid bacteria; Milchwissenschaft, 40 (9): 518-520 (1985).

3. Then one proceeds to thoroughly mix the inoculum and the medium with a movement that is, for example, first rotational and then horizontal and vertical translational.

4. The plating by pouring can be done in a simple layer (for microaerophilic microorganisms) or in a double layer (for anaerobic microorganisms); in the latter case, once the first layer has solidified, another $6 \pm 1$ ml, for example, of agarized medium is added.

5. Incubate the dishes upside down, for example at 37 $\pm$ 1 °C for 48-72 hours under conditions of anaerobiosis (Gas Pack containers + kit for anaerobiosis). As regards the methods for using the anaerobiosis system, follow the instructions enclosed in the kit.

6. The evaluation of the result is made by counting the colonies of bacteria that have grown in the dish.
Since the number of microorganisms present in the sample is not known a priori, one proceeds, for example, as follows:

For example, when one has an idea, albeit approximate, as to the number of microorganisms present in the sample, at least 2 successive decimal dilutions are inoculated (e.g.: $10^{-8}$, $10^9$) with the assumption that the dilutions contain a number of cells comprised, for example, between 10 and 300.

For example, when one has no idea of the number of microorganisms present in the sample, it is necessary to inoculate more than 3 successive decimal dilutions (e.g.: $10^5$, $10^{-6}$, $10^{-7}$, $10^{-8}$, $10^{-9}$, $10^{-10}$), then considering only the ones which give rise to a number of colonies comprised, for example, between 10 and 300.

By proceeding in the above-described manner, one obtains a growth of microorganisms such as to be able to determine their number by means of a bacterial count.

For the count of the genus *Lactobacillus,* the reference methods used are:
FIL IDF 149A:1997, FIL-IDF 122C:1996, EN-ISO 6887-1 For the count of the genus *Bifidobacterium* the reference methods are:
Bulletin of the IDF 252 (3.1.2.iv and viii), EN-ISO 6887-1

Subsequently, one proceeds to calculate the results.

The result, expressed as Colony Forming Units (CFU), is obtained with the formula:

$$[c] = \Sigma C / [n_1 + 0.1\, n_2)] \times D$$

where:
$\Sigma C$ is the sum of the colonies counted in all the dishes, $n_1$ is the number of dishes counted in the first dilution, $n_2$ is the number of dishes counted in the second dilution, and D is the dilution from which the first counts were obtained and takes into account all the dilutions made, also considering the primary dilution.

With the method used it is possible to determine the total bacterial count [c] = [g] + [b] after a process of microencapsulation of the bacteria with a lipid matrix. It should be noted that the value [g] represents the "total count of bacteria actually coated or encapsulated" following the encapsulation/coating process.

Therefore, the % of coated bacteria = ([g] / [c]) x100= (([c] - [b]) / [c])x100 can be determined given that the value [b], which represents the count of uncoated bacteria per gram of sample after a process of encapsulation of the microorganisms with a lipid matrix, is determined using a saline solution in accordance with known methods. The value [b] comprises the free uncoated cells and/or partially coated cells.

To determine the count (number of bacteria present) of a "naked" lyophilizate, also called starting sample [a], or to determine the value of [b] the method described below can be used.

One proceeds to prepare the first dilution of the sample to be analysed, which can be a sample of "naked" lyophilizate [a] or a sample [b] containing the uncoated and/or partially coated cells.

Preferably, one may proceed as described below.

1. Allow the test sample to thermostat at a temperature of 25$\pm$2 °C for a time, for example, of from 1 to 2 hours, if it was stored in a freezer, or for at least 30 minutes, if it was stored at a temperature comprised, for example, between 3 and 6 °C.

2. Weigh about 3-4 g of lyophilizate in a sterile Stomacher bag.

3. Add the peptonated saline solution (prepared as indicated in standard UNI EN ISO 6887-1) in such a way as to obtain a first 1:10 dilution of the sample. The peptonated saline solution has a pre-sterilisation pH of 7 $\pm$ 0.2 at 25 °C and is as described above.

The subsequent steps, namely, homogenisation of the sample, preparation of the successive decimal dilutions (serial dilution), inoculation of the sample and calculation and expression of the results are carried out as described above for the count of an encapsulated or coated bacterial lyophilizate [c].

The method enables the determination of the % of bacterial survival in a sample, for example in a lyophilized sample, obtained with an encapsulation or coating process. The % of bacterial survival allows the yield of said process to be evaluated.

With the value [c], determined thanks to the present method, and having the value [a] and of the dilution factor (d), it is possible to determine the % of survival = {([coated bacteria] + [uncoated bacteria])/viable count of "naked" sample x dilution factor} x 100; in the formula:

$$\{ ([c]/[a] x [d]) x100 \}.$$

From an experimental point of view, it was observed that a double coating of the cells ensures better sealing of the bacteria from the environment, producing a continuous film without pores communicating with the outside.

For example, a reference sample consists of a bacterial lyophilizate prepared as follows. In practical terms, a hydrogenated palm fat, Tm = 60 °C (commercial name Revel C), and a glyceryl dipalmitostearate, with Tm= 57-60 °C (commercial name Precirol Ato 5), were sprayed in succession onto lyophilized bacteria so as to give a double coating. The first fat and the second fat were applied in a ratio by weight of 3:1.

Following the encapsulation process the evaluation of the bacterial viability of an encapsulated lyophilizate requires a pre-treatment using a dilution with a solution containing a buffer, for example borate buffer, capable of creating openings in the lipid coating such as the render the bacteria available for the normal procedure of analysis of the bacterial count.

Example of procedure for carrying out the successive decimal dilutions.

a.1 For the preparation of the successive decimal dilutions, use sterile test tubes filled with 9 ml of peptonated saline solution, prepared as above.

a.2 Transfer, by means of a sterile pipette, 1 ml of the primary dilution ($1x10^{-1}$ for all types of samples, or K x $10^2$ in the case of sachets) into a test tube containing 9 ml of sterile diluent as per step a.1, avoiding contact between the pipette and the diluent.

In this manner the primary dilution will be diluted 10 times, thus obtaining, respectively, the $1x10^{-2}$ dilutions for all types of sample, or K x $10^{-3}$ in the case of sachets.

a.3 When transferring the primary dilution into the diluent, avoid introducing the pipette further than 1 cm into the primary dilution itself and try to make the primary dilution flow completely into the diluent, expelling and aspirating 3 consecutive times (rinsing) a quantity of liquid equal to about 1 ml.

a.4 Thoroughly mix the new dilution by pressing the test tube into the recess provided in a vortex-type mechanical mixer. The test tube should be held between the thumb and index finger at about 1-1.5 cm from the top, so as to ensure that a vortex forms to nearly the full height of the test tube itself, without, however, wetting the cap.

Correct mixing is obtained by carrying out a 2-step cycle 3 times: agitation with vortex for 2-3 seconds and a pause of about 1 second.

a.5 Prepare the successive serial decimal dilutions as per step a.2, transferring 1 ml of the last dilution prepared into 9 ml of diluent.

a.6 Use a new sterile pipette for each dilution.

a.7 Continue preparing the serial dilutions until judging to have obtained one containing an appropriate number of microorganisms, i.e. comprised between 10 and 300 / ml.

a.8 Distribute 1 ml of the dilution judged appropriate into a Petri dish, also inoculating the most concentrated and the most diluted serial decimal dilutions.

If no idea is had as to the number of microencapsulated lactic bacteria and/or bifidobacteria contained in the sample, it will be necessary to inoculate a number of successive decimal dilutions (e.g.: $10^{-5}$, $10^{-6}$, $10^{-7}$, $10^{-8}$, $10^{-9}$, $10^{-10}$), then considering in the analysis of the results only the ones that give rise to a number of colonies comprised between 10 and 300.

a.9 Add $12\pm1$ ml of LAPTg agar, allowed to stabilise at a temperature of $45\pm 0.5$ °C, to each inoculated dish.

a.10 The maximum time which may elapse between the dissolving of the sample and the addition of the culture medium to the Petri dish must not be greater than 15 minutes.

a.11 Mix the culture medium and dilution of the sample in a uniform manner first with rotational movements and then horizontal and vertical translational movements.

a.12 Allow to solidify.

a.13 Add a second layer of the same LAPTg agar ($6 \pm 1$ ml) at a temperature of $45\pm 0.5$ °C, distributing it in such a way as to uniformly cover the underlying layer; then allow to solidify.

a.14 Incubate the dishes upside down at 37 ± 0.5 °C for 48-72h under conditions of anaerobiosis (Gas Pack jars + kit for anaerobiosis). As regards the methods for using the anaerobiosis system, follow the instructions enclosed in the kit.

The incubation time depends on the state of vitality of the bacteria contained in the sample and is therefore decided, case by case, after observing the diameter of the colonies after 48 hours of incubation; in the case of slow growth, the incubation should be extended to 72 hours.

The results are calculated and expressed counting only the dishes containing a number of colonies comprised between 10 and 300. The result will be expressed as CFU, i.e. Colony-Forming Units.

Calculate the result using the following formula:

$$[c] = \Sigma C / (n1 + 0.1\ n2)D$$

where:

ΣC    is the sum of the colonies counted in all the dishes
n1    is the number of dishes counted in the first dilution
n2    is the number of dishes counted in the second dilution

d is the dilution from which the first counts were obtained and takes into account all the dilutions made, also considering the primary dilution.

Example:

Dishes of the $10^{-8}$ dilution : 250-238
Dishes of the $10^{-9}$ dilution : 23-26

Result:

$$\frac{250 + 238 + 23 + 26}{(2 + 0.1x2) \cdot 10^{-8}} = 244 \cdot 10^{8}\ \text{CFU/g,}$$

or per sachet.

Round the result obtained to three significant digits. If the decimal digit to be rounded is 5, round up to the next integer. The result of the example given will thus be $24.4 \cdot 10^{-9}$ CFU/g, or per sachet.

The result obtained represents the total count of the microencapsulated lactic bacteria and bifidobacteria present in the sample analysed. The titre is expressed in CFU/gram for the samples represented by "hard gelatin capsules", "tablets", "soft gel" and "products NOT packaged in doses or units". The titre is expressed in CFU/X gram sachet for the samples represented by "sachets". In the latter case it is possible to obtain the count per gram of product (CFU/g) by dividing the result obtained by the weight in grams (X) of the sachet.

**Claims**

1. A food product selected from the group consisting of milk, cheese, butter, margarine, yoghurt and cream wherein said product comprises probiotic bacteria selected from the group consisting of species: *L. acidophilus, L. crispatus, L. gasseri, L. delbrueckii, L. salivarius, L. casei, L. paracasei, L. plantarum, L. rhamnosus, L. reuteri, L. brevis, L. buchneri, L. fermentum, B. adolescentis, B. angulatum, B. bifidum, B. breve, B. catenulatum, B. infantis, B. lactis,*

*B. longum, B. pseudocatenulatum, S. thermophilus,* and wherein said probiotic bacteria are coated with at least one lipid coating of vegetable origin, and said at least one lipid coating consists of polyglyceryl-6-distearate.

2. The product according to claim 1, wherein said probiotic bacteria are coated with a single lipid coating consisting of polyglyceryl-6-distearate.

3. The product according to any one of claims 1-2, wherein the weight ratio [bacteria: lipid coating] is 50:50 or 40:60.

4. Milk comprising probiotic bacteria, wherein said probiotic bacteria are coated with at least one lipid coating in accordance with any one of claims 1-3.

5. Use of probiotic bacteria which are coated with a single lipid coating in accordance with any one of claims 1-3, for the preparation of a food product selected from group consisting of milk, cheese, butter, margarine, yoghurt and cream.

6. Use of polyglyceryl-6-distearate in a method for preparing a food product according to any one of claims 1-3.

**Patentansprüche**

1. Nahrungsmittelprodukt, ausgewählt aus der Gruppe bestehend aus Milch, Käse, Butter, Margarine, Joghurt und Sahne, wobei das Produkt probiotische Bakterien, ausgewählt aus der Gruppe bestehend aus den Arten: L. acidophilus, L. crispatus, L. gasseri, L. delbrueckii, L. salivarius, L. casei, L. paracasei, L. plantarum, L. rhamnosus, L. reuteri, L. brevis, L. buchneri, L. fermentum, B. adolescentis, B. angulatum, B. bifidum. B. breve, B. catenulatum, B. infantis, B. lactis, B. longum, B. pseudocatenulatum und S. thermophilus, umfasst und wobei die probiotischen Bakterien mit mindestens einer Lipidbeschichtung pflanzlichen Ursprungs überzogen sind und die mindestens eine Lipidbeschichtung aus Polyglyceryl-6-distearat besteht.

2. Produkt gemäß Anspruch 1, wobei die probiotischen Bakterien mit einer einzelnen Lipidbeschichtung, die aus Polyglyceryl-6-distearat besteht, überzogen sind.

3. Produkt gemäß einem der Ansprüche 1 bis 2, wobei das Gewichtsverhältnis [Bakterien : Lipidbeschichtung] 50:50 oder 40:60 ist.

4. Milch, die probiotische Bakterien umfasst, wobei die probiotischen Bakterien mit mindestens einer Lipidbeschichtung in Übereinstimmung mit einem der Ansprüche 1 bis 3 überzogen sind.

5. Verwendung von probiotischen Bakterien, die mit einer einzelnen Lipidbeschichtung in Übereinstimmung mit einem der Ansprüche 1 bis 3 überzogen sind, für die Herstellung eines Nahrungsmittelprodukts, das aus der Gruppe bestehend aus Milch, Käse, Butter, Margarine, Joghurt und Sahne ausgewählt ist.

6. Verwendung von Polyglyceryl-6-distearat in einem Verfahren zur Herstellung eines Nahrungsmittelprodukts gemäß einem der Ansprüche 1 bis 3.

**Revendications**

1. Produit alimentaire choisi dans le groupe consistant en le lait, le fromage, le beurre, la margarine, le yaourt et la crème où ledit produit comprend des bactéries probiotiques choisies dans le groupe consistant en les espèces: *L. acidophilus, L. crispatus, L. gasseri, L. delbrueckü, L. salivarius, L. casei, L. paracasei, L. plantarum, L. rhamnosus, L. reuteri, L. brevis, L. buchneri, L. fermentum, B. adolescentis, B. angulatum, B. bifidum, B. breve, B. catenulatum, B. infantis, B. lactis, B. longum, B. pseudocatenulatum, S. thermophilus,* et où lesdites bactéries probiotiques sont revêtues d'au moins un revêtement lipidique d'origine végétale, et ledit au moins un revêtement lipidique consiste en polyglycéryl-6-distéarate.

2. Produit selon la revendication 1 où lesdites bactéries probiotiques sont revêtues d'un seul revêtement lipidique consistant en polyglycéryl-6-distéarate.

**3.** Produit selon l'une quelconque des revendications 1-2 où le rapport en poids [bactéries: revêtement lipidique] est 50:50 ou 40:60.

**4.** Lait comprenant des bactéries probiotiques où lesdites bactéries probiotiques sont revêtues d'au moins un revêtement lipidique selon l'une quelconque des revendications 1-3.

**5.** Utilisation de bactéries probiotiques qui sont revêtues d'un seul revêtement lipidique selon l'une quelconque des revendications 1-3 pour la préparation d'un produit alimentaire choisi dans le groupe consistant en le lait, le fromage, le beurre, la margarine, le yaourt et la crème.

**6.** Utilisation du polyglycéryl-6-distéarate dans un procédé pour préparer un produit alimentaire selon l'une quelconque des revendications 1-3.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4332790 A
- US 2007098847 A
- US 2005002874 A
- US 2003109025 A
- JP H08242763 B

### Non-patent literature cited in the description

- **JI-KANG JUNG et al.** Survival of double-microencapsulated Bifidobacterium breve in milk in simulated gastric and small intestinal conditions. *Database accession no. FS-2007-08-Pa2013 & JOURNAL OF FOOD SCIENCE AND NUTRITION,* 2007, vol. 12 (1), 58
- **SIUTA-CRUCE P et al.** IMPROVING PROBIOTIC SURVIVAL RATES MICROENCAPSULATION PRESERVES THE POTENCY OF PROBIOTIC MICROORGANISMS IN FOOD SYSTEMS. *FOOD TECHNOLOGY, INSTITUTE OF FOOD TECHNOLOGISTS, CHICAGO, IL, US,* 01 October 2001, vol. 55 (10), 36, 38-4042
- **DEMAN, JC ; ROGOSA M ; SHARPE ME.** *J. Applied Microbiology,* 1960, vol. 23, 130
- **ROGOSA M ; MITCHELL JA ; WISEMAN RF.** *J. Bacteriology,* 1951, vol. 62, 132
- **FONT DE VALDEZ, G.** Influence of the recovery medium on the viability of injured freeze-dried lactic acid bacteria. *Milchwissenschaft,* 1985, vol. 40 (9), 518-520